# EUROPEAN PATENT SPECIFICATION

(11) **EP 3 377 017 B1**
(45) Date of publication and mention of the grant of the patent: **31.07.2019**
(21) Application number: 16784995.9
(22) Date of filing: 10.08.2016
(51) Int. Cl.: A61G 1/02, A61B 6/04, A61G 7/012, A61G 1/048

(54) **DEVICE FOR TRANSPORT AND MEDICAL CARE OF PATIENTS**
VORRICHTUNG ZUM TRANSPORT UND ZUR MEDIZINISCHEN VERSORGUNG VON PATIENTEN
DISPOSITIF DE TRANSPORT ET DE SOINS MÉDICAUX DE PATIENTS

(30) Priority: 16.11.2015 SI 201500273
(43) Date of publication of application: 26.09.2018
(73) Proprietor: Novak M D.o.o., 1218 Komenda (SI)
(72) Inventor: NOVAK, Miha, 1218 Komenda (SI)
(74) Representative: Jersan, Tatjana
(86) International application number: PCT/SI2016/000018
(87) International publication number: WO 2017/086882

(56) References cited:
- DE-A1- 2 321 968
- US-A- 3 814 414
- US-B1- 6 421 854

## Description

The invention refers to a device for transport and medical care of patients.

The international classification of patents for inventions classifies a solution of this type to the field of human necessities, namely to the field of medicine and within the framework of the latter to the devices for accommodation or transport specially adapted for patients or disabled persons, and particularly to devices for transport and medical care of patients that are known to persons skilled in the art under the English word *stretcher.*

The underlying problem of the invention is, on the one hand, how to conceive a device that will allow a rapid, simple, safe and reliable transport of a patient over a solid surface and in terms of regular use also on ramps from an ambulance to an emergency department, and on the other hand, to examine a patient without any need for the patient being moved or turned, especially by using radiography. In said context the term "vehicles" denotes not only vehicles for locomotion by land but also includes means for locomotion by air and water, while the term "emergency department" represents any location, at which a transported patient will be given medical care, especially medical examination and radiography.

A medical stretcher is known from AU 2015100190 B4. A device of this type comprises a chassis with wheels and a patient support which is movable in vertical direction by means of a relatively complicated mechanism and, if necessary, also detachable from said chassis. In principle, a device of this type allows a rapid, safe and reliable transport of a patient over a solid surface and in terms of regular use also on ramps, namely from an ambulance to an emergency department; however, said device is rather heavy due to said properties and requires plenty of effort from paramedics, while, on the other hand, said mechanism of the device makes access of an X-ray device very difficult, wherein a patient often needs to be turned during the radiography - depending on the injury - and this fact is linked to a risk of inadvertent additional injuries and/or other medical complications. Moreover, metallic elements of the frame or chassis of the support prevent a free use of an X-ray apparatus, since these parts are also visible in an X-ray image - instead of a patient's organism parts.

A similar yet simpler and presumably easier to handle is a device known from a flysheet of the manufacturer Stryker, 3800 E Centre Ave., Portage. MI 49002 which is currently available in the market under the brand name *Stryker*®*.* A device of this type is also known from US 6 421 854 B1 and also comprises a chassis with wheels arranged in corners of a virtual parallelogram, and a bed supported by means of telescopic, vertically extensible support posts on said chassis. Said support posts are arranged at a mutual distance and are both on a line of a longitudinal axis of symmetry of said chassis. The bed consists of a frame including longitudinal and transversal metallic elements. A combination of said arrangement of support posts and presence of said reinforcing elements in said bed prevents access of an X-ray device during radiography without a need of turning a patient on the one hand, while said reinforcing elements are also visible in X-ray images on the other hand and make a preliminary examination of a patient more difficult and this is of key importance in emergency cases and decisive for an efficient further medical care of a patient.

In fact, the device for transport and medical care of patients of the invention consists of a chassis with wheels arranged in corners of a virtual parallelogram with a central longitudinal axis of symmetry, and of a bed comprising a support frame that is supported on said chassis by means of a pair of telescopic support posts mutually spaced in direction of said axis and vertically displaceable. The invention foresees said telescopic support posts to be arranged each at its side of said axis, namely each in its diagonally opposite corner of said virtual parallelogram formed by the wheels of the chassis. The invention further foresees said support frame of said bed to be without any transversal or longitudinal reinforcing element, with the exception of areas diagonally opposite with respect to said longitudinal axis that are reasonably present above said support posts. At a side facing away from the support, said frame of the bed is provided with panels from a non-metallic material that are adapted to receive a stretcher cushion.

The invention will be explained in more detail in the continuation by way of an embodiment illustrated in the enclosed drawing, in which
Fig. 1 shows a device for transport and medical care of patients in isometry;
Fig. 2 shows a device from Fig. 1 while in use;
Fig. 3 shows a chassis of the device from Fig. 1, in isometry as well;

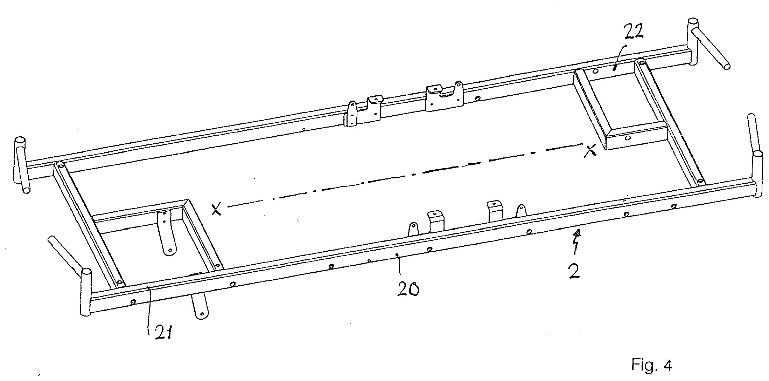

Fig. 4 shows a frame of a bed of the device from Fig S1. 1, in isometry as well;

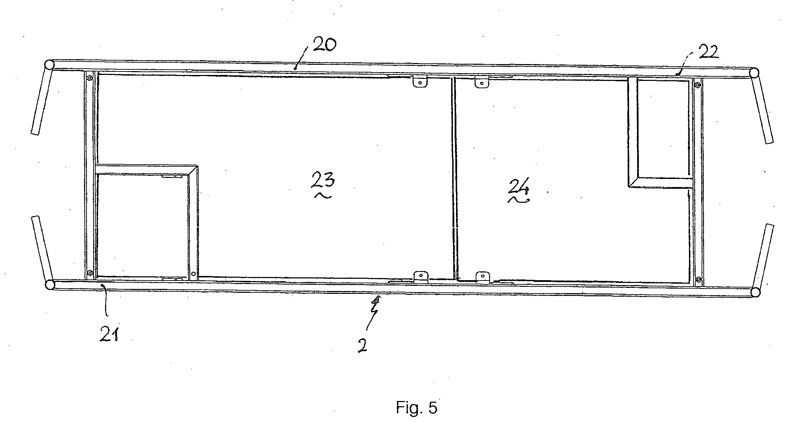

Fig. 5 shows the device from Fig S1. 1, a bottom view.

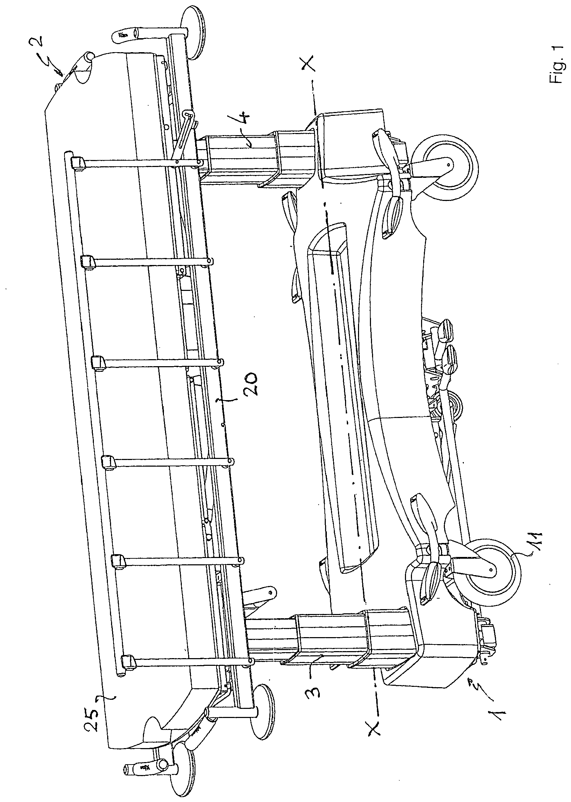

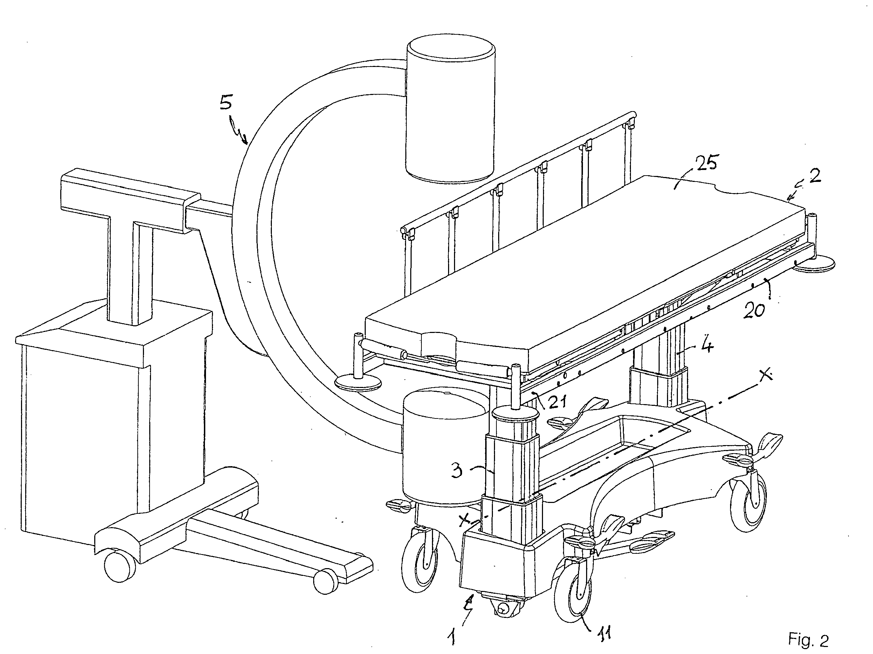

A device for transport and medical care of patients *per se* is shown in Fig. 1, while Fig. 2 shows it in a combination with an apparatus 5 for performing medical examination of patients by radiography. Said apparatus 5 comprises a C-shaped frame that allows performing examination on a patient in a way understandable to a person skilled in the art without a need of moving or turning the patient.

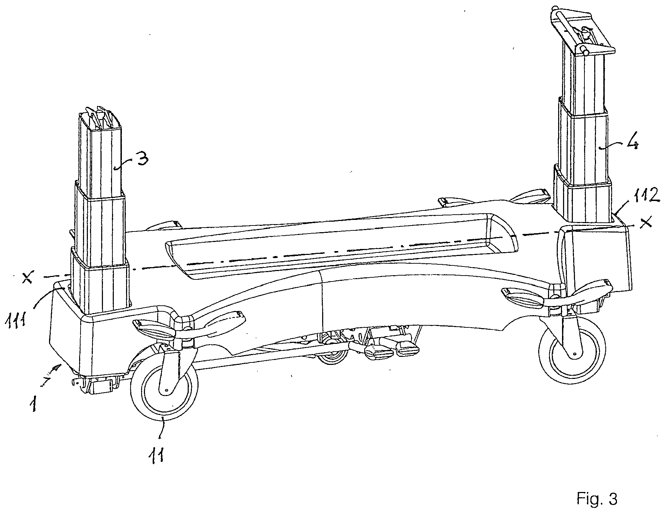

The device for transport and medical care of patients consists of a chassis 1 with wheels 11 arranged in corners of a virtual parallelogram with a central longitudinal axis of symmetry X-X (Figs. 1-3).

The device further comprises a bed 2 that comprises a support frame 20 and is supported on said chassis 1 by means of a pair of telescopic support poles 3, 4 spaced apart in direction of said axis X-X and displaceable in vertical direction.

Said telescopic support poles 3, 4 are arranged each at its side of said axis X-X, more particularly each in its diagonally opposite corner 111, 112 of said virtual parallelogram formed by the wheels 11 of the chassis 1. Such design allows a very efficient care of a patient and offers a possibility of a rapid and free access with the apparatus 5 for performing X-ray examinations from one or the other side of said longitudinal axis X-X of the device of the invention. Moreover, the support frame 20 of said bed 2 is formed without any transversal or longitudinal reinforcing element that could be visible on images obtained by the apparatus 5 for performing X-ray examinations instead of the examined parts of a patient's body, with the exception of areas 21, 22 that are reasonably present above the support poles 3, 4 and are diagonally opposite to said axis X-X.

For the same reason, the frame 20 of the bed 2 is provided at a side facing away from said support frame by panels 23, 24 from a non-metallic material, said panels being adapted to receive a stretcher cushion 25 and to allow the X-rays to pass, unlike the metallic reinforcing elements. The panels 23, 24 are practically invisible on images received from the apparatus 5 for performing X-ray examinations and therefore allow an unrestricted, efficient and credible examination of parts of a patient's body in the framework of known methods and procedures from the specialist area and prior art.

## Claims

1. A device for transport and medical care of patients , the device consisting of:
a chassis (1) with wheels (11) arranged in corners of a virtual parallelogram with a central longitudinal axis of symmetry (X-X),
a bed (2) comprising a support frame (20), said support frame (20) of said bed (2) being formed without any transversal or longitudinal reinforcing element, with the exception of areas (21, 22) that are reasonably present above the support poles (3, 4) and are diagonally opposite to said central longitudinal axis of symmetry (X-X),
a pair of telescopic support poles (3, 4) mutually spaced apart in direction of said axis (X-X) and displaceable in vertical direction, said pair of telescopic support poles (3, 4) supporting said bed (2) on said chassis (1),
**characterized in that** said telescopic support poles (3, 4) are each arranged at its side of said axis (X-X), namely each in its diagonally opposite corner (111, 112) of said virtual parallelogram formed by the wheels (11) of the chassis (1).

2. The device according to claim 1 **characterized in that** the support frame (20) of the bed (2) is provided at a side facing away from said support frame (20) with panels (23, 24) from a non-metallic material adapted to receive a stretcher cushion (25).

## Patentansprüche

1. Vorrichtung zum Transport und zur medizinischen Versorgung von Patienten, bestehend aus: einem Fahrgestell (1) mit, in den Ecken eines virtuellen Parallelogramms angeordneten Rädern (11), mit einer zentralen Längs-Symmetrieachse (X-X), einer Liege (2), die einen Stützrahmen (20) umfasst, wobei der Stützrahmen (20) der Liege (2) keine Quer- oder Längsversteifungselemente aufweist, mit Ausnahme der Bereiche (21, 22), die sich oberhalb der Stützstangen (3, 4) befinden und der zentralen Längs-Symmetrieachse (X-X) diagonal gegenüberliegen, einem Paar von Teleskop-Stützstangen (3, 4), die in Richtung dieser Achse (X-X) voneinander beabstandet und in vertikaler Richtung verschiebbar sind, wobei das Paar Teleskopstützstangen (3, 4) die Liege (2) auf dem Fahrgestell (1) stützt, **dadurch gekennzeichnet, dass** die Teleskopstützstangen (3, 4) jeweils auf einer Seite der genannten Achse (X-X) angeordnet sind, und zwar jeweils in einer diagonal gegenüberliegenden Ecke (111, 112) des durch die Räder (11) des Fahrgestells (1) gebildeten virtuellen Parallelogramms.

2. Vorrichtung nach Anspruch 1, **dadurch gekennzeichnet, dass** der Stützrahmen (20) der Liege (2) an einer, dem Stützrahmen (20) abgewandten Seite Paneele (23, 24) aus einem nichtmetallischen Material aufweist, die zur Aufnahme eines Kissens (25) der Tragebestimmt sind.

## Revendications

1. Dispositif de transport et de soins médicaux de patients, le dispositif comprenant : un châssis (1) avec des roues (11) disposées dans des coins d'un parallélogramme virtuel avec un axe longitudinal central de symétrie (X-X), un lit (2) comprenant un cadre de support (20), ledit cadre du support (20) dudit lit (2) étant formé sans aucun élément de renforcement transversal ou longitudinal, à l'exception de zones (21, 22) qui sont raisonnablement présentes au-dessus des montants de support (3, 4) et sont diagonalement opposées audit axe central longitudinal de symétrie (X-X), une paire de montants de support télescopiques (3, 4) mutuellement espacés en direction dudit axe (X-X) et mobiles en direction verticale, ladite paire de montants de support télescopiques (3, 4) supportant ledit lit (2) sur ledit châssis (1), **caractérisé en ce que** ledits montants de support télescopiques (3, 4) sont chacun disposés sur son côté dudit axe (X-X), à savoir chacun dans son coin diagonalement opposé (111, 112) dudit parallélogramme virtuel formé par les roues (11) du châssis (1).

2. Dispositif selon la revendication 1 **caractérisé en ce que** le cadre de support (20) du lit (2) est disposé sur un côté opposé audit cadre de support (20) avec des panneaux (23, 24) à partir d'un matériau non métallique conçu pour recevoir un coussin de civière (25).
